# EUROPEAN PATENT APPLICATION

(11) **EP 4 555 941 A2**
(43) Date of publication of application: **21.05.2025**
(21) Application number: 24213746.1
(22) Date of filing: 18.11.2024
(51) Int. Cl.: A61B 10/02, A61B 90/30

(54) **ILLUMINATION ASSEMBLY, GEAR SHIFTING ASSEMBLY, AND ELECTRIC TUBE CUTTING NEEDLE**

(30) Priority: 20.11.2023 CN 202311543525; 20.11.2023 CN 202311542435; 20.11.2023 CN 202311543655; 20.11.2023 CN 202311542273
(71) Applicant: Suzhou Leapmed Healthcare Corporation, Suzhou, Jiangsu 215163 (CN)
(72) Inventor: JIANG, Xinhua, Suzhou, 215163 (CN); WANG, Qin, Suzhou, 215163 (CN); WANG, Baozhong, Suzhou, 215163 (CN); ZHU, Yu, Suzhou, 215163 (CN); GU, Wei, Suzhou, 215163 (CN)
(74) Representative: Gill Jennings & Every LLP

(57) **Abstract**

The present application proposes an illumination assembly, a gear shifting assembly, an electric tube cutting needle. The electric tube cutting needle defines a front end and a rear end of the travel of a winding slider in the winding assembly by means of a detection module and a control module, and achieves an electrified winding operation and excitation reset operation by means of a motor drive module which drives the winding assembly and the excitation assembly, which can ensure that the winding slider stops when the backward movement reaches the rear end during the winding process, as well as that the winding slider stops when the forward movement reaches the front end during the reset process. Compared with the conventional manual tube cutting needles that require manual winding operation, it is more labour-saving and stable, and can improve the efficiency and convenience of the operation of the tube cutting needle.

## Description

### Technical Field

The present application relates to the technical field of medical devices and, in particular, to an illumination assembly, a gear shifting assembly, and an electric tube cutting needle.

### Background

Puncture biopsy is the primary method of obtaining a histopathological diagnosis for bone and soft tissue tumours. Puncture biopsy needles are generally used under ultrasound guidance and in conjunction with a puncture frame. An ultrasound image serves as a visual guide to assist a surgeon in inserting a biopsy needle into the vicinity of the tumour tissue that needs to be sampled, and then the biopsy needle is excited to remove the tissue from the tumour. The puncture frame can assist a surgeon in keeping the biopsy needle in an ultrasound plane, so that the biopsy needle is always displayed in a line shape on the ultrasound image. Puncture biopsy procedures are divided into lateral cutting and tube cutting. Tube cutting is made by cutting off and extracting the tissue directly from an outer needle. Compared to biopsy needles for lateral cutting, tube cutting needles can extract fuller tissue.

The puncture biopsy needles for tube cutting include an inner needle, an outer needle, and a cut-off needle, and the inner needle, outer needle, and cut-off needle are coaxially nested from the inside to the outside in sequence. The direction of puncture needle feed is defined as 'front' and the direction opposite to the direction of puncture needle feed is defined as 'rear'. The principle of tube cutting for puncture biopsy is as follows: 1. the tip of the inner needle is inserted into the tissue; 2. the outer needle and the cut-off needle are excited, the needle end of the outer needle is in the front, the needle end of the cut-off needle is in the rear, and the outer needle protrudes beyond the tip of the inner needle and cuts into the tissue by means of a cutting edge, and at this time, the tissue enters into the outer needle; 3. after the outer needle stops, the cut-off needle continues to move forward, and an elastic cut-off piece on the tip of the cut-off needle protrudes through a through hole on the portion of the outer needle that is close to the tip into the outer needle to cut off the tissue inside the outer needle; 4. finally, the inner needle, the outer needle, and the cutting-off needle for tube cutting are pulled out of the body, the cut-off needle is removed, and then the tissue inside the outer needle is pushed out through the inner needle.

The inventor has found in the process of realizing the present invention that the electric tube cutting needles of the prior art have at least the following problems: the operating room for ultrasonic puncture is usually dark, which affects the accuracy of the operation; in addition, it is easy for a user to accidentally touch a power switch when operating a button or pushbutton on a tube cutting needle; a gear shifting block is worn out due to repeated impacts and there is a risk of damage and malfunctioning; manual winding can cause hand fatigue for an operator and reduce the stability of subsequent surgical operations; the accuracy of the cut-off piece of the cut-off needle entering the through hole of the outer needle needs to be improved; sometimes there may be situations where the tissue cannot be cut off well, which needs to be improved.

### Summary

The present application proposes an illumination assembly, a gear shifting assembly, and an electric tube cutting needle, which can solve the above-mentioned problems in the prior art. Other beneficial effects will be described in conjunction with specific embodiments.

According to an aspect of an exemplary embodiment of the present application, there is provided an illumination assembly for an electric tube cutting needle, the electric tube cutting needle comprising a puncture biopsy needle assembly, a battery, a motor, a housing, a first pushbutton for the excitation of a puncture needle, and a winding button for winding, the puncture biopsy needle assembly piercing from a first end of the housing, wherein the illumination assembly comprises an illumination lamp, a circuit board, and a power switch, wherein:
the housing is formed by an upper housing and a lower housing snapped together, and extends as a whole in the needle feed direction, and the illumination lamp is located at the first end of the housing and is electrically connected to the circuit board;
the first pushbutton is located at a bottom of the upper housing and close to the first end of the housing, and the winding button is located at a first side of the housing and close to the first end of the housing, wherein the first side of the housing is formed by split-joining of a first side of the upper housing and a first side of the lower housing;
the power switch is located on a second side of the housing and close to a second end of the housing, wherein the second side of the housing is a side opposite to the first side of the housing, and the second end of the housing is an end opposite to the first end of the housing.

According to an aspect of an exemplary embodiment of the present application, optionally, the battery is arranged such that its axis is parallel to the needle feed direction;
a plurality of ribs are provided in the position in an inner wall of the bottom of the lower housing that corresponds to the battery, and the battery is positioned on the plurality of ribs so as to allow the presence of a passage between the battery and the inner wall of the lower housing;
the circuit board fits against the inner wall of the first side of the housing and is close to the first end of the housing, and a wire leading from the circuit board extends fitting against the inner wall of the first side of the housing to a position flush with the power switch in the needle feed direction, and then is connected to the power switch through the passage.

According to an aspect of an exemplary embodiment of the present application, optionally, the power switch has a main body and a toggle block protruding from the main body;
the main body is positioned within the housing of the electric tube cutting needle;
the housing is provided with an aperture, in which the toggle block is positioned, and an end face of the free end of the toggle block is flush with the surface of the housing.

According to an aspect of an exemplary embodiment of the present application, optionally, the illumination lamp comprises a lamp cylinder and a lamp bead , wherein:
the axis of the lamp cylinder is parallel to the needle feed direction, a side surface of the lamp cylinder is connected to the circuit board, and an end face of the opening of the lamp cylinder is flush with a surface of the first end of the housing;
the lamp bead is fixed inside the lamp cylinder.

According to yet another aspect of an exemplary embodiment of the present application, there is provided a gear shifting assembly for an electric tube cutting needle, comprising a gear shifting block and a second pushbutton, wherein:
the gear shifting block comprises a blocking hook, a base plate and a slide groove,
the blocking hook comprises a first arm, a second arm, a third arm, a fourth arm, and a connecting portion;
the base plate is connected to and coplanar with the bottom of the slide groove;
the first arm is connected to the second arm in an L-shape, the second arm is connected to the top edge of the first wall of the slide groove, and the second arm extends in the same direction as the first wall;
the third arm is connected to the fourth arm in an L-shape, the fourth arm is connected to the top edge of the second wall of the slide groove, and the second arm extends in the same direction as the second wall;
two ends of the connecting portion are connected to the junction between the first arm and the second arm and the junction between the third arm and the fourth arm respectively, so that the connecting portion, the first arm and the third arm form a U-shape, the first arm and the third arm extending in a direction away from the base plate; and
the second pushbutton is provided on a lower surface of the base plate, and the pressing direction of the second pushbutton is the same as the extension direction of the first arm and the third arm.

According to yet another aspect of an exemplary embodiment of the present application, optionally, the gear shifting block further comprises a recess for accommodating the travel of the cut-off needle seat;
the slide groove is provided with a stopper therein, which is adjacent to the end of the recess that is located inside the slide groove, so that the side of the stopper that faces an inlet of the slide groove is used to receive the impact from the cut-off needle seat when travelling.

According to yet another aspect of an exemplary embodiment of the present application, optionally, a first protrusion is provided at the junction between the first arm and the second arm;
a second protrusion is provided at the junction between the third arm and the fourth arm; and
the first protrusion and the second protrusion both protrude towards the base plate.

According to yet another aspect of an exemplary embodiment of the present application, optionally, wall grooves are provided on the outer sides of the first wall and the second wall of the slide groove, the wall grooves extending in a direction perpendicular to the bottom of the slide groove.

According to yet another aspect of an exemplary embodiment of the present application, optionally, the thickness of the first wall and the second wall of the slide groove is 4 to 6 times the thickness of the bottom of the slide groove.

According to a further aspect of an exemplary embodiment of the present application, an electric tube cutting needle is provided, comprising: a housing, a tube cutting needle mechanical unit and a tube cutting needle control unit, wherein the interior of the housing has a first track and a second track arranged in parallel;
the tube cutting needle mechanical unit comprises:
   a puncture biopsy needle assembly comprising an inner needle, an outer needle, and a cut-off needle;
   a needle seat component comprising a cut-off needle seat, an outer needle seat and an inner needle seat disposed on the second track, the cut-off needle seat, the outer needle seat and the inner needle seat being connected to the inner needle, the outer needle and the cut-off needle, respectively, and the cut-off needle seat, the outer needle seat and the inner needle seat being distanced from a needle exit end sequentially from near to far, the inner needle seat being fixedly connected to the housing, a spring being provided between the inner needle seat and the outer needle seat;
   a winding assembly for winding the needle seat component, comprising a winding slider disposed on the first track, the winding slider being provided so as to be capable of driving the cut-off needle seat and the outer needle seat in the direction opposite to the needle feed direction to a winding position;
   an excitation assembly for excitation of the cut-off needle seat and the outer needle seat in the winding position;
   a gear shifting assembly for adjusting an excitation gear of the electric tube cutting needle;
the tube cutting needle control unit comprises:
   a motor drive module for driving the winding assembly and the excitation assembly to realize winding and excitation reset;
   a detection module for detecting the position of the winding slider;
   a control module for controlling the operation of the motor drive module according to the detection result of the detection module.

According to a further aspect of an exemplary embodiment of the present application, optionally, the winding assembly is arranged such that:
when the winding slider is in a first position, the winding slider can be moved in the needle feed direction under the drive by the motor drive module, such that a connection hole at the end of the winding slider snaps together with the second protrusion at the end of the cut-off needle seat;
when the winding slider is moved to a second position, the winding slider can be moved in the direction opposite to the needle feed direction under the drive by the motor drive module, such that the cut-off needle seat and the outer needle seat are moved along with the winding slider in the direction opposite to the needle feed direction and such that the spring is compressed to store energy;
when the winding slider is again in the first position, the winding slider stops moving.

According to a further aspect of an exemplary embodiment of the present application, optionally, the gear shifting assembly is a gear shifting assembly according to an exemplary embodiment of the present application mentioned above.

According to a further aspect of an exemplary embodiment of the present application, optionally, the excitation assembly comprises: a trigger linkage, a trigger button disposed at the rear end of the trigger linkage, and a first pushbutton connected to the trigger linkage, wherein
when the trigger button is pressed or the first pushbutton is pushed, the trigger linkage is moved in the needle feed direction, and the end of the trigger linkage is moved along a ramp-like surface of an unlocking bevel on the cut-off needle seat, thereby separating the winding slide from the cut-off needle seat; then the cut-off needle seat and the outer needle seat are moved in the needle feed direction under the action of the elastic force of the spring; the outer needle seat stops moving when it touches the gear shifting block, the cut-off needle seat continues moving under the action of inertia and stops when it touches the gear shifting block, and at the same time, the cut-off needle seat locking hook on the cut-off needle seat and the gear shifting block locking hook on the gear shifting block snap together with each other.

According to a further aspect of an exemplary embodiment of the present application, optionally, the motor drive module comprises a motor and a screw rod, wherein the motor is provided to be capable of performing forward or reverse rotation, thereby driving the screw rod to perform forward or reverse rotation, which in turn pushes the winding slider to move in the needle feed direction or in the direction opposite to the needle feed direction.

According to a further aspect of an exemplary embodiment of the present application, optionally, the detection module comprises: a front contact switch and a rear contact switch, wherein
the rear contact switch is arranged to emit a first detection signal when the rear contact switch comes into contact with the first protrusion on the winding slider;
the front contact switch is arranged to emit a second detection signal when the front contact switch comes into contact with the first protrusion on the winding slider.

According to a further aspect of an exemplary embodiment of the present application, optionally, the control module comprises a circuit board, the circuit board being arranged such that:
if the circuit board receives the first detection signal, the circuit board controls the motor drive module to drive the winding slider to move in the needle feed direction;
if the circuit board receives the second detection signal, the circuit board controls the motor drive module to drive the winding slider to move in the direction opposite to the needle feed direction.

According to a further aspect of an exemplary embodiment of the present application, optionally, the electric tube cutting needle further comprises an illumination assembly according to an exemplary embodiment of the present application mentioned above.

According to a further aspect of an exemplary embodiment of the present application, optionally, the cut-off needle seat has a groove body recessed downwardly from its upper surface, the groove body extends along a longitudinal direction, which is the direction of travel of the cut-off needle seat, and the transverse end face of the groove body that is close to the outer needle seat forms a blocking surface;
a longitudinally extending limit lever is connected to the side of the outer needle seat that is close to the cut-off needle seat, and a limit block is connected to the end of the limit lever, the limit block being unable to rotate about the longitudinal direction taken as an axis within the groove body, thereby constituting a circumferential limit;
a portion of the limit lever and the limit block are located within the groove body, the limit block has a width greater than the width of the limit lever, and the limit lever has such a length that while the extension of the cut-off needle seat impacts the gear shifting block of the electric tube cutting needle, the side of the limit block that faces the outer needle seat abuts against the blocking surface.

According to a further aspect of an exemplary embodiment of the present application, optionally, the electric tube cutting needle further comprises a needle seat spring, which is arranged to be sleeved on the cut-off needle and stuck between the cut-off needle seat and the outer needle seat; the needle seat spring is a compression spring having an elastic force less than that of the spring that provides excitation for the electric tube cutting needle.

According to a further aspect of an exemplary embodiment of the present application, optionally, the end of the extension of the cut-off needle seat that faces the outer needle seat is provided with a notch, and the first end of the needle seat spring abuts against an end face of the notch, the end face being perpendicular to the cut-off needle;
the lower surface of the outer needle seat has a downwardly extending convex rib, and the second end of the needle seat spring abuts against the side surface of the convex rib that faces the cut-off needle seat.

According to the technical solutions of the present application, by providing an illumination lamp in the electric tube cutting needle, it is convenient for the surgeons' surgical operation, and the power switch is designed to be far away from other buttons or pushbuttons, so that it is not easy to be touched by mistake.

According to the technical solutions of the present application, by designing the gear shifting assembly with a blocking hook having a U-shaped structure, it enhances the stability and is more resistant to impact; by adding a stopper in the slide groove, it helps to improve the ability of the slide groove to withstand impact; and by providing grooves on the outer sides of the two walls of the slide groove, it allows the slide groove to have a transverse deformation, so as to avoid shattering when impacted. These designs enable the electric tube cutting needle to have high mechanical strength, not easy to break, and improve the overall reliability.

According to the technical solutions of the present application, by means of the detection module and the control module defining the front end and the rear end of the travel of the winding slider in the winding assembly, as well as by means of the motor drive module driving the winding assembly and the excitation assembly to achieve the electrified winding operation and the excitation reset operation, it is possible to ensure that the winding slider stops when the backward movement reaches the rear end during the winding process, as well as that the winding slider stops when the forward movement reaches the front end during the reset process. Compared with the conventional manual tube cutting needles that require manual winding operation, it is more labour-saving and stable, and can improve the efficiency and convenience of the operation of the tube cutting needle.

According to the technical solutions of the present application, by designing the limit lever and the limit block, the cut-off needle seat can accurately enter the through hole of the outer needle, and the length of the entry is more precise. This combination structure helps to improve the accuracy of the length of the cut-off piece of the cut-off needle entering the outer needle, thus helping to enable the length of the cut-off piece entering the outer needle to precisely meet the required reasonable length. At the same time, the restriction block cannot rotate in the groove body, which makes it difficult to produce mutual misalignment between the outer needle and the cut-off needle in the circumferential direction, thereby helping to align the cut-off piece with the side hole on the outer needle. As a result, the cut-off precision and stability of the electric tube cutting needle are improved.

According to the technical solutions of the present application, by installing a needle seat spring between the cut-off needle seat and the outer needle seat, a cut-off force can be additionally provided to the cut-off needle, which helps to ensure that the tissue can be cut off. At the same time, the needle seat spring has a certain buffering effect, can absorb part of the axial vibration of the cut-off needle, improve the stability of the cut-off needle, so as to improve the overall stability of the needle tip to a certain extent, and reduce the needle tip jitter. The cut-off structure also helps to improve the reliability of the tissue cut-off, and has good practicality.

### Brief Description of the Drawings

For purposes of illustration and not limitation, the present invention will now be described in accordance with preferred embodiments of the present invention, with particular reference to the accompanying drawings, in which:
FIGS. 1A and 1B are schematic diagrams of the external structure of an electric tube cutting needle according to an exemplary embodiment of the present invention;
FIG. 2 is a schematic diagram of the internal structure of an electric tube cutting needle according to an exemplary embodiment of the present invention, wherein the individual structures are shown to be positioned within the housing on one side;
FIG. 3 is a partial schematic diagram of the internal structure of an electric tube cutting needle according to an exemplary embodiment of the present invention;
FIG. 4 is a structural schematic diagram of a motor drive module of an electric tube cutting needle according to an exemplary embodiment of the present application;
FIG. 5 is a structural schematic diagram of a screw rod according to an exemplary embodiment of the present application;
FIG. 6 is an exploded view of a screw rod according to an exemplary embodiment of the present application;
FIG. 7 is a structural schematic diagram of the assembly of a winding slider, a cut-off needle seat and a gear shifting block according to an exemplary embodiment of the present application;
FIG. 8 is a schematic diagram of a state of the electric tube cutting needle when winding begins according to an exemplary embodiment of the present application;
FIG. 9 is a schematic diagram of a state of the electric tube cutting needle when winding is completed according to an exemplary embodiment of the present application;
FIG. 10 is an exploded view of the assembly body of a winding slider, a cut-off needle seat and a gear shifting block from one perspective according to an exemplary embodiment of the present application;
FIG. 11 is an exploded view of the assembly body of a winding slider, a cut-off needle seat and a gear shifting block from another perspective according to an exemplary embodiment of the present application;
FIG. 12 is an exploded view of the assembly body of a winding slider, a cut-off needle seat, a trigger linkage, a gear shifting block according to an exemplary embodiment of the present application;
FIG. 13 is a view observed from the back side shown in FIG. 12;
FIG. 14 is a structural schematic diagram of a trigger linkage according to an exemplary embodiment of the present application;
FIG. 15 is a schematic diagram of a state of an electric tube cutting needle after excitation according to an exemplary embodiment of the present application;
FIG. 16 is a structural schematic diagram of a gear shifting block according to an exemplary embodiment of the present application;
FIG. 17 is a structural schematic diagram in which a winding slider, a cut-off needle seat, and an outer needle seat are positioned on a slide track according to an exemplary embodiment of the present application;
FIG. 18 is a structural schematic diagram of a slide track on a housing according to an exemplary embodiment of the present application;
FIG. 19 is a structural schematic diagram of a slide track on a housing from another perspective according to an exemplary embodiment of the present application;
FIG. 20 is a diagram of the positions of a cut-off needle seat and an outer needle seat after excitation of an electric tube cutting needle according to an exemplary embodiment of the present application;
FIG. 21 is a diagram of the positions of a cut-off needle seat and an outer needle seat after winding of an electric tube cutting needle according to an exemplary embodiment of the present application;
FIG. 22 is a structural schematic diagram of the connection of a gear shifting block with a housing of an electric tube cutting needle according to an exemplary embodiment of the present application;
FIG. 23 is a structural schematic diagram of a part of the housing which is connected to a gear shifting block of an electric tube cutting needle according to an exemplary embodiment of the present application;
FIG. 24 is a structural schematic diagram of a gear shifting block of an electric tube cutting needle from another perspective according to an exemplary embodiment of the present application;
FIG. 25 is a top view of the connection of a gear shifting block with an outer needle seat of an electric tube cutting needle according to an exemplary embodiment of the present application;
FIG. 26 is a stereoscopic view of the connection of a gear shifting block and an outer needle seat of an electric tube cutting needle according to an exemplary embodiment of the present application;
FIG. 27 is a stereoscopic view of the connection of a gear shifting block and an outer needle seat of an electric tube cutting needle from another perspective according to an exemplary embodiment of the present application;
FIG. 28 is a schematic diagram of the external structure of an electric tube cutting needle according to another exemplary embodiment of the present application;
FIG. 29 is a side schematic diagram of the external structure of an electric tube cutting needle according to another exemplary embodiment of the present application;
FIG. 30 is a sectional view of an electric tube cutting needle according to another exemplary embodiment of the present application shown in FIG. 28;
FIG. 31 is a structural schematic diagram of a gear shifting block according to another exemplary embodiment of the present application;
FIG. 32 is a schematic diagram of an outer needle and part of a tip of a cut-off needle of an electric tube cutting needle according to an exemplary embodiment of the present application;
FIG. 33 is a schematic diagram of the structure of a cut-off needle seat of an electric tube cutting needle according to another exemplary embodiment of the present application;
FIG. 34 is a schematic diagram of the structure of an outer needle seat of an electric tube cutting needle according to another exemplary embodiment of the present application;
FIG. 35 is a schematic diagram of a combined structure formed by a cut-off needle seat and an outer needle seat of an electric tube cutting needle according to an exemplary embodiment of the present application;
FIG. 36 is a schematic diagram of a spring provided between a cut-off needle seat and an outer needle seat of an electric tube cutting needle according to an exemplary embodiment of the present application.

### Detailed Description

In the embodiments of the present application, a spring is added between a cut-off needle seat and an outer needle seat, which can additionally provide a cut-off force to the cut-off needle and helps to ensure that the tissue can be cut off. A detailed description is provided below.

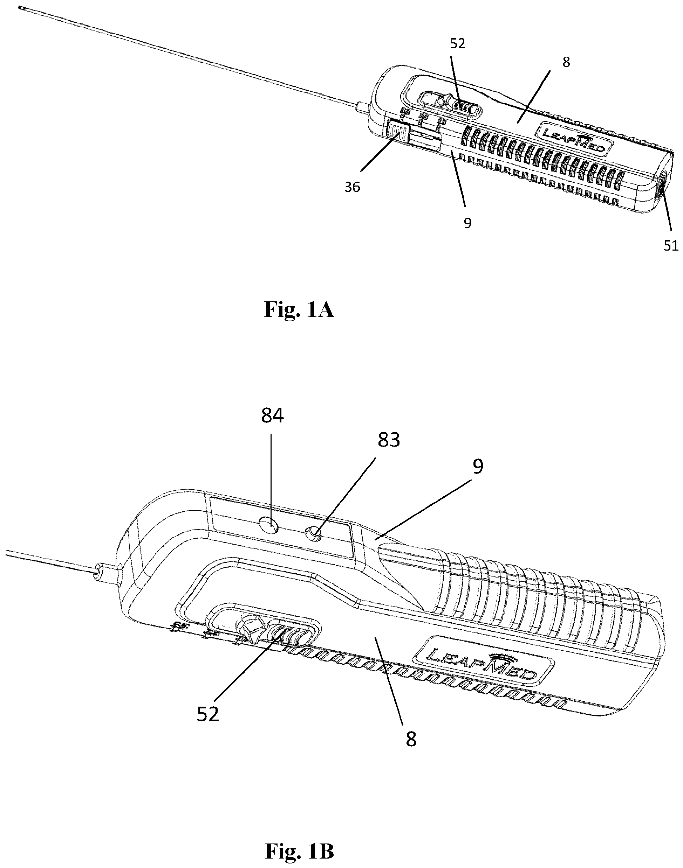

FIGS. 1A and 1B are schematic diagrams of the external structure of an electric tube cutting needle according to an exemplary embodiment of the present invention. As shown in FIGS. 1A and 1B, the electric tube cutting needle comprises an upper housing 8 and a lower housing 9 as well as a puncture biopsy needle assembly (comprising an inner needle, an outer needle, and a cut-off needle). The housing is provided with a trigger button 51 and a first pushbutton 52 for the excitation of a puncture needle and a second pushbutton 36 for adjusting the excitation gear of the puncture needle. The butting surface of the upper housing 8 with the lower housing 9 is provided with a display hole 84 from which an indicator lamp 82 can be observed, and a hole from which a winding button 83 is allowed to protrude out of the housing.

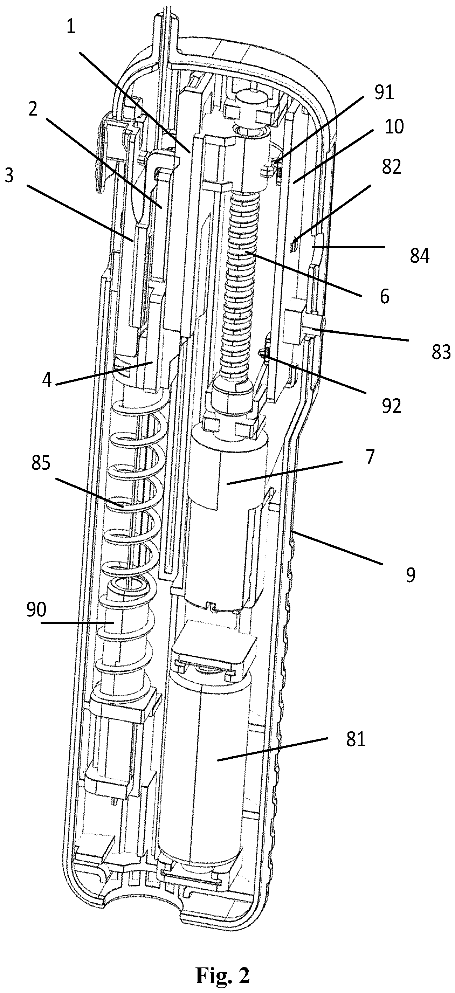

FIG. 2 is a diagram of the overall internal layout of an electric tube cutting needle according to an exemplary embodiment of the present application, the electric tube cutting needle comprising a motor drive module, a winding\excitation assembly, and the like, wherein the motor drive module and the winding\excitation assembly are all located inside the housing. The lower housing 9 can be shown and the upper housing 8 can be omitted in FIG. 2. The motor drive module comprises a motor 7, a screw rod 6, a front contact switch 91, a rear contact switch 92, a battery 81, a winding button 83, and an indicator lamp 82. The mounting position of the indicator lamp 82 corresponds to a display hole 84 in the housing. The winding\excitation assembly comprises a winding slider 1, a cut-off needle seat 2, an outer needle seat 4, a gear shifting block 3, an inner needle seat 90, and a spring 85. The spring 85 is arranged between the outer needle seat 4 and the inner needle seat 90, and the inner needle seat 90 is fixedly connected to the lower housing 9. The motor drive module drives the winding\excitation assembly to achieve the functions of winding, excitation, resetting and the like. The technical solutions of the embodiments of the present application are described in detail below.

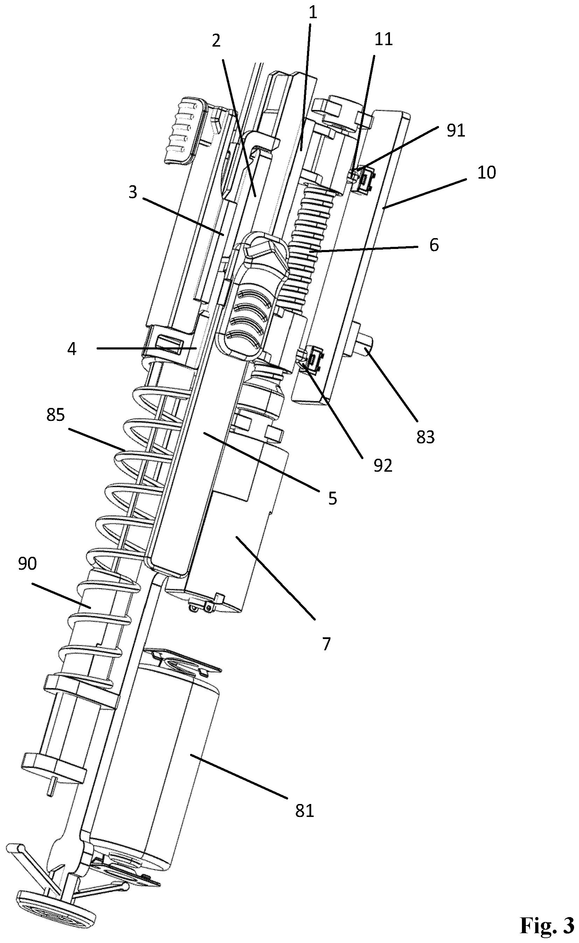

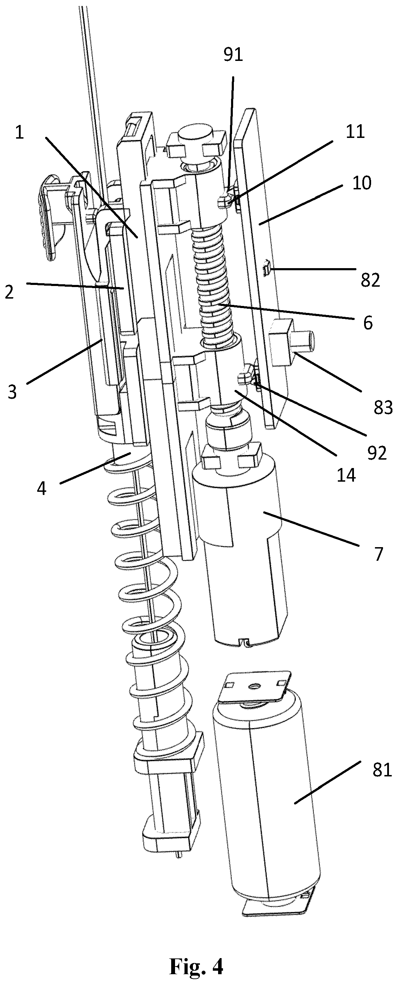

FIGS. 3 and 4 show the internal structure of the electric tube cutting needle of the exemplary embodiment of the present application. FIGS. 3 and 4 are illustrated from different angles, and for the sake of clarity of the illustration, the trigger linkage 5 is omitted from FIG. 4. The electric tube cutting needle according to an embodiment of the present application is driven by a motor to wind up, and thus is operated more effortlessly. The motor 7 of the motor drive module drives a screw rod 6, which passes through a threaded hole in the inner surface of a tubular structure 14 on the side of the winding slider 1 that is away from the cut-off needle seat 2, so as to be able to drive the winding slider 1 to slide backwards and forwards along a track located on the inner side of the housing, the backwards and forwards sliding direction being parallel to the direction of the needle feed during the puncture operation. FIGS. 3 and 4 show the winding slider 1 at two positions to indicate the two endpoint positions of its sliding, and in the actual structure, only one winding slider 1 is provided still as shown in FIG. 2.

The winding slider 1 drives the cut-off needle seat 2 of the electric tube cutting needle to move, and the cut-off needle seat 2 can push the outer needle seat 4 when moving.

The cut-off needle seat 2, the winding slider 1, and a circuit board 10 are arranged sequentially side by side in the direction perpendicular to the needle feed direction. The circuit board 10 is provided with a front contact switch 91 and a rear contact switch 92 on the side thereof that is close to the winding slider 1. A first protrusion 11 on the tubular structure 14 touches the front contact switch 91 and the rear contact switch 92 respectively when the winding slider 1 is moved forward and backward so as to cause the two switches to produce action. The circuit board 10 is provided with a winding button 83 and an indicator lamp 82, which is used to display the operating state of the battery 81 or the connection state with a power supply. As shown in FIG. 2, the winding button 83 protrudes from a hole in the housing. The motor 7 is connected to the battery 81, and the motor 7 can also be connected to an external low-voltage DC power supply.

The front contact switch 91 and the rear contact switch 92 serve to determine the position of the winding slider 1. Other position sensing elements can also be selected as the contact switches.

In the embodiments of the present application, for example, when the winding slider 1 is in the starting position, the first protrusion 11 rests against the rear contact switch 92, and when the winding button 83 is pressed, the motor 7 rotates so as to drive the screw rod 6 to rotate and push the winding slider 1 forward (which is a reset process). After the first protrusion 11 on the surface of the winding slider 1 touches the front contact switch 91, the motor 7 rotates in the reverse direction, and pushes the winding slider 1 backward through the transmission of the screw rod 6 (which is a winding process), and the motor 7 stops until the first protrusion 11 touches the rear contact switch 92. The motor 7 is caused to run through the action generated by the front contact switch 91 and the rear contact switch 92, so that the winding slider 1 is moved back and forth, thereby realizing the actions of resetting and winding.

In the embodiments of the present application, before pressing the winding button 83 and moving the winding slider 1 from back to front to reset, the cut-off needle seat 2 and the outer needle seat 4 have been reset after the completion of the excitation by means of a thrust from the elastic force of the spring 85. During winding, the winding slider 1 are snapped together with the cut-off needle seat 2, and the winding slider 1 drives the cut-off needle seat 2 to move backward. The position of the front contact switch 91 is arranged such that the winding slider 1 can be moved to the reset position.

FIG. 4 shows the winding slider 1 at two positions so as to show two states of the winding slider 1, i.e. a first state in which the winding slider 1 is located in a first position in the rear and a second state in which the winding slider 1 is located in a second position in the front. After pressing the winding button 83, the winding slider 1 is moved from the first state to the second state to the first state again, and then waits for an excitation operation.

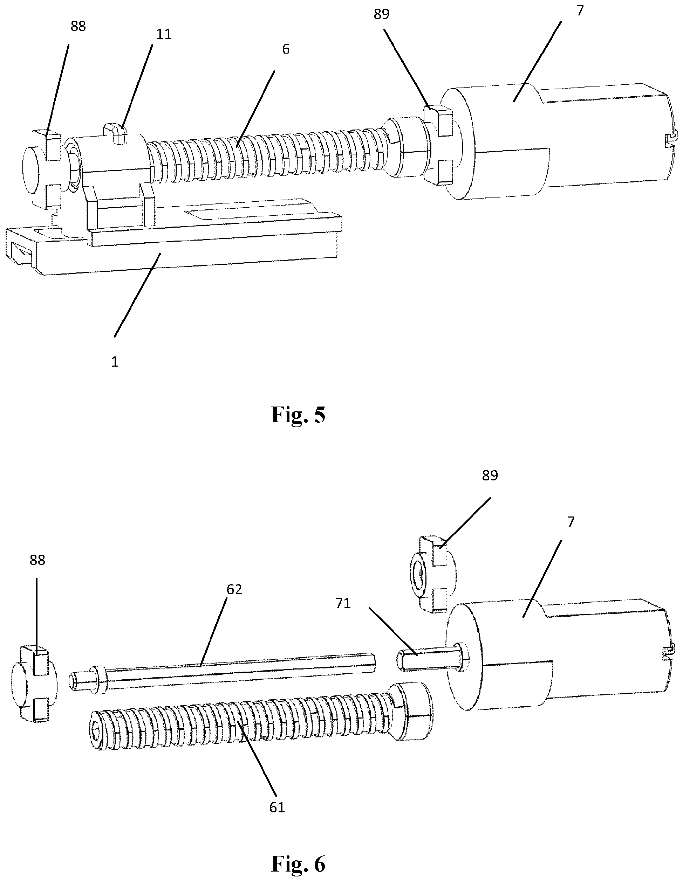

FIGS. 5 and 6 show the structure of a screw rod according to an exemplary embodiment of the present application. As shown in FIGS. 5 and 6, the screw rod 6 comprises a hollow plastic screw rod 61 and a metal shaft 62 filled in the middle of the plastic screw rod 61. The metal shaft 62 is used to increase the strength of the screw rod 6.

The metal shaft 62 protrudes from the first end of the plastic screw rod 61, and the protruding end is provided with a first bearing 88 fixedly arranged on the housing. The second end of the plastic screw rod 61 is sleeved on a rotary shaft 71 of the motor 7. The second end of the screw rod 6 is provided with a second bearing 89 fixedly arranged on the housing. The second bearing 89 can also be arranged on the rotary shaft 71 of the motor 7. The screw rod 6 is rotationally arranged on the housing by two bearings, so that the accuracy can be ensured.

It is also possible to provide a bearing at the end of the screw rod that is away from the motor separately and not provide a bearing at the end connected to the motor. In this way, however, the force at the end of the screw rod that is close to the motor is all borne by the motor, which will affect the coaxiality of the screw rod.

In the embodiments of the present application, the bearings are respectively arranged on the metal shaft that is away from the motor end and on the rotary shaft of the motor, which can realize the connection of a rotary shaft made of metal with a bearing.

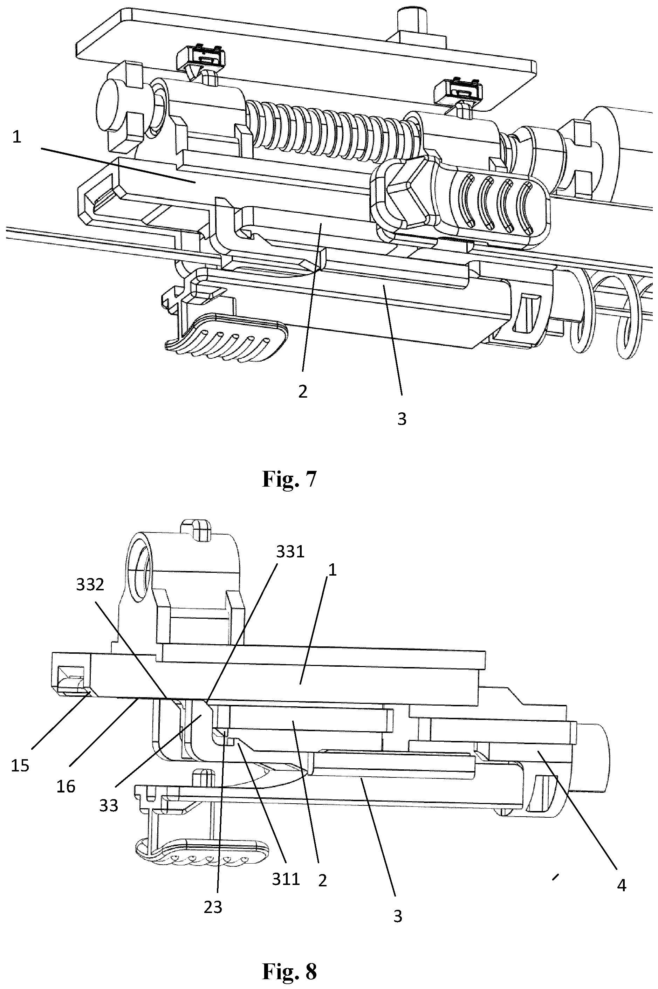

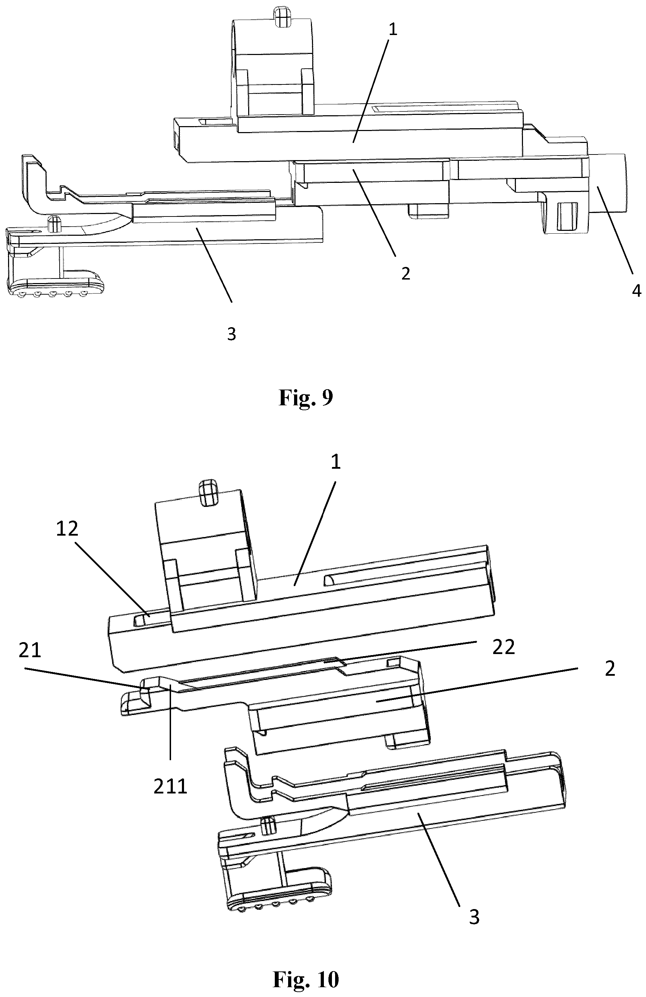

The winding and excitation assembly is further described below in conjunction with the accompanying drawings. The first step of the winding and excitation reset operation is winding. Referring to FIGS. 7, 8, and 9, FIG. 7 is a structural schematic diagram of the assembly of the winding slider 1, the cut-off needle seat 2, and the gear shifting block 3 according to an exemplary embodiment of the present application. In FIG. 7, the winding slider 1 drives the cut-off needle seat 2 and the gear shifting block 3 to be connected together. In FIG. 7, two winding sliders 1 can be seen, and this merely represents two positions of one winding slider 1, as in FIGS. 3 and 4. FIG. 8 shows the state of each component at the start of winding, and during winding, except for the gear shifting block 3, the winding slider 1, the cut-off needle seat 2 and the outer needle seat 4 in the figure are moved together in the direction opposite to the needle feed direction. FIG. 9 shows the state of each component when the winding is completed.

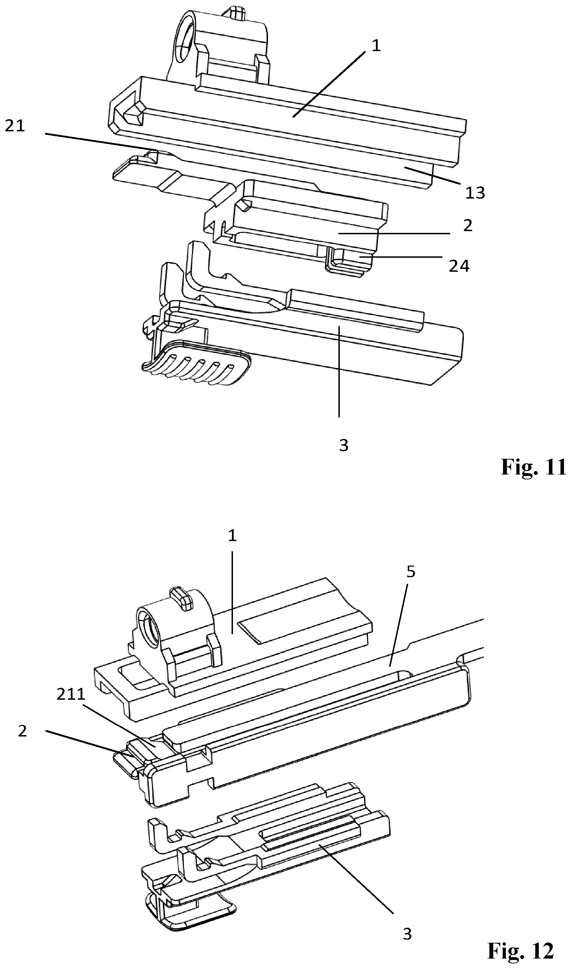

Referring to FIGS. 10 and 11, FIGS. 10 and 11 are exploded views of the assembly of the winding slider 1, the cut-off needle seat 2 and the gear shifting block 3 from different perspectives according to an embodiment of the present application. The winding slider 1 and the cut-off needle seat 2 are connected in a snapping manner. The front end of the surface of the cut-off needle seat 2 that is connected to the winding slider 1 is provided with a second protrusion 21. The winding slider 1 is provided with a corresponding connecting hole 12. The second protrusion 21 protrudes into the connecting hole 12 to realize the snapping, so that the winding slider 1 can drive the cut-off needle seat 2 to move in the direction opposite to the needle feed direction. The connecting hole 12 may be a through hole or a blind hole, and is a through hole in FIG. 10. In FIG. 10, the front side surface of the second protrusion 21 is a vertical surface, and the rear side surface is an inclined surface that is inclined outwardly, which is an unlocking bevel 211.

During winding, after the connection hole 12 in the winding slider 1 hooks the second protrusion 21 on the cut-off needle seat 2, the winding slider 1 is moved in the direction opposite to the needle feed direction. In conjunction with FIGS. 7 to 9, the cut-off needle seat 2 and the outer needle seat 4 abut against, and then slid together in the direction opposite to the needle feed direction, and the spring 85 between the outer needle seat 4 and the inner needle seat 90 is compressed to store energy. When the winding slider 1 is moved to a designated position and then is locked motionless, the winding action is completed.

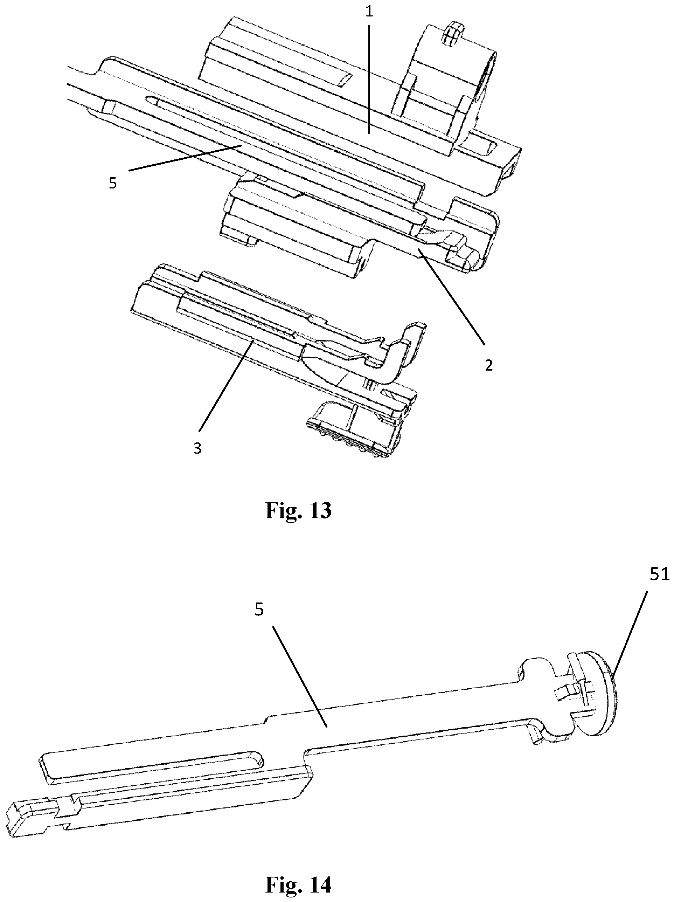

The second step of the operation is excitation. FIGS. 12 and 13 give the position of the trigger linkage 5, and FIG. 14 is a structural schematic diagram of the trigger linkage of an embodiment of the present application. Referring to FIG. 12, when the trigger linkage 5 is moved in the needle feed direction, its end is moved along the ramp-like surface of the unlocking bevel 211, topping off the winding slide 1 and the cut-off needle seat 2.

As shown in FIG. 11, the part of the winding slider 1 that is connected to the cut-off needle seat 2 is provided with a first longitudinal groove 13. As shown in Figure 10, a second longitudinal groove 22 is provided in the middle of the right side of the unlocking bevel 211 on the cut-off needle seat 2, wherein the end of the trigger linkage 5 in the needle feed direction is located in the second longitudinal groove 22; the first longitudinal groove 13 is arranged opposite to the second longitudinal groove 22, and the groove wall of the second longitudinal groove 22 is located inside the first longitudinal groove 13. As shown in FIG. 12, in the starting state, the front end of the trigger linkage 5 is located in the rear end in the second longitudinal groove 22, at a certain distance from the second protrusion 21. In conjunction with FIG. 1, the trigger linkage 5 is pushed in the needle feed direction by pressing the trigger button 51 at the rear end of the trigger linkage 5 or by pushing the first pushbutton 52 connected with the trigger linkage 5 and arranged in the middle of the housing, and the connection hole 12 in the winding slider 1 is disengaged from the second projection 21 on the cut-off needle seat 2 by means of the unlocking bevel 211. The spring 85 between the outer needle seat 4 and the inner needle seat 90 is released and then pushes the outer needle seat 4 and the cut-off needle seat 2 together in the needle feed direction. In this case, the outer needle and the cut-off needle are moved in the needle feed direction at the same time. In the biopsy surgical conditions, the outer needle protrudes beyond the tip of the inner needle and cuts the tissue by means of a cutting edge, and the tissue enters into the outer needle. The outer needle seat 4 is moved in the need feed direction and stops after impacting the rear lateral portion of the gear shifting block 3. The cut-off needle seat 2 continues to move for a certain distance in the needle feed direction under the action of inertia after the outer needle seat 4 stops, and stops after impacting the gear shifting block 3 and at the same time is locked by the locking hook structure between the cut-off needle seat 2 and the gear shifting block 3 (which locking hook structure comprises a cut-off needle seat locking hook 23 and a gear shifting block locking hook 311). In this case, a cut-off piece with elasticity on the tip of the cut-off needle protrudes into the outer needle through a through hole in the part of the outer needle that is close to the tip and cuts off the tissue inside the outer needle.

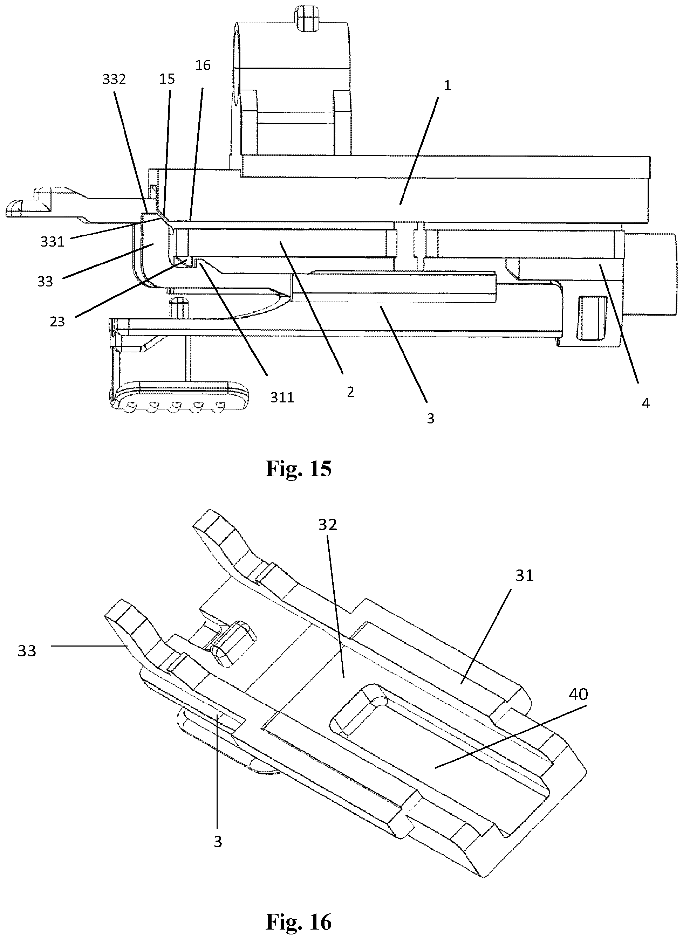

The state of the electric tube cutting needle after excitation is as shown in FIG. 15. Referring to FIG. 15, the gear shifting block 3 is sleeved on the bottoms of the cut-off needle seat 2 and the outer needle seat 4. In conjunction with FIG. 16, the left and right sides on the top of the gear shifting block 3 are respectively provided with a blocking wall 31 which is parallel to the needle feed direction, and between the blocking walls 31 is a slide groove 32. Referring to FIG. 15, the cut-off needle seat 2 and the outer needle seat 4 are provided with respective rectangular tabs at the bottoms thereof, and the rectangular tabs can be moved within the slide groove 32. The front end of the bottom of the winding slider 1 is provided with a downward-pressing bevel 15. The front end of the blocking wall 31 of the gear shifting block 3 is curved upward to form a blocking hook 33 perpendicular to the needle feed direction. The side of the end of the blocking hook 33 that is away from the needle feed direction is provided with a blocking hook bevel 331 corresponding to the downward-pressing bevel 15. An upwardly protruding gear shifting block locking hook 311 is provided at the position on the blocking wall 31 that is close to the blocking hook 33. The side surface of the gear shifting block locking hook 311 on the side of the needle feed direction is a vertical surface, and the other side of the gear shifting block locking hook 311 is an inclined surface which is outwardly inclined. A cut-off needle seat locking hook 23 that protrudes toward the gear shifting block 3 is provided in the corresponding position on the cut-off needle seat 2. The side surface of the cut-off needle seat locking hook 23 on the side of the needle feed direction is an inclined surface that is outwardly inclined, and the side surface on the other side is a vertical surface. Before the blocking hook 33 is pressed down, the cut-off needle seat locking hook 23 and the gear shifting block locking hook 311 are located in a straight line that is parallel to the needle feed direction so that they are hooked to each other.

In FIG. 15, the winding slider 1 is moved in the needle feed direction, the gear shifting block 3 is pressed in the direction away from the winding slider 1 (i.e., below in FIG. 15) by means of the downward-pressing bevel 15 of the winding slider 1, and the gear shifting block locking hook 311 and the cut-off needle seat locking hook 23 are disengaged from each other in the state as shown in FIG. 8. After the winding slider 1 continues moving in the needle feed direction, the locking hooks (311, 23) between the cut-off needle seat 2 and the gear shifting block 3 remain in a disengaged state. After the winding slider 1 continues moving in the needle feed direction to the front contact switch 91, the winding action is repeated. The connection hole 12 in the winding slider 1 hooks the second protrusion 21 on the cut-off needle seat 2 and is moved in the direction opposite to the needle feed direction. In this case, the cut-off piece 041 is disengaged from the through hole of the outer needle 03 that is close to the tip. The cut-off needle seat 2 is moved to abut against the outer needle seat 4 and then they together slide in the direction opposite to the needle feed direction. The spring 85 between the outer needle seat 4 and the inner needle seat 90 is compressed to store energy, at which time the inner needle protrudes from the outer needle 03. If tissue is taken inside the outer needle 03, the inner needle will push out the taken tissue.

It is to be noted that in FIGS. 2-4 and 7, the states of the gear shifting block locking hook 311, the cut-off needle seat locking hook 23, the downward-pressing bevel 15, and the blocking hook bevel 331 should be as shown in FIG. 8, i.e., a top surface 332 of the blocking hook 33 abuts against a bottom edge 16 of the winding slider 1, and the gear shifting blocklocking hook 311 and the cut-off needle seat locking hook 23 are kept in a disengaged state.

When the winding slider 1 is moved to the designated position and then is locked motionless, the winding action is completed and the next excitation biopsy action can be performed.

The structure of the winding and excitation assembly is described above, and the working process of winding and excitation reset is as follows:
At the start of winding, the cut-off needle seat 2 and the outer needle seat 4 are in an excited state, and the winding slider 1 is located in a first position at the rear. When the winding button 83 is pressed, the winding slider 1 is moved in the needle feed direction, the downward-pressing bevel 15 presses the blocking hook 33 in the direction away from the winding slider 1, and the cut-off needle seat locking hook 23 is disengaged from the clamping with the gear shifting block locking hook 311. The winding slider 1 continues to move in the needle feed direction, the connection hole 12 at the end of the winding slider 1 is snapped with the second protrusion 21 at the end of the cut-off needle seat 2, and the first protrusion 11 of the winding slider 1 is in contact with the front contact switch 91, i.e., the winding slider 1 is located in the second position in the front. The winding slider 1 drives the cut-off needle seat 2 to move together in the direction opposite to the needle feed direction. After the cut-off needle seat 2 touches the outer needle seat 4, the winding slider 1, the cut-off needle seat 2 and the outer needle seat 4 are moved together in the direction opposite to the needle feed direction. When the first protrusion 11 of the winding slider 1 contacts the rear contact switch 92, the winding slider 1 stops moving, and the spring 85 between the outer needle seat 4 and the inner needle seat 90 is compressed.

Upon excitation, the trigger linkage 5 is moved in the needle feed direction along the ramp of the unlocking bevel 211 to cause the winding slider 1 to be separated from the cut-off needle seat 2. The cut-off needle seat 2 and the outer needle seat 4 are moved in the needle feed direction under the action of the elastic force of the spring 85. The outer needle seat 4 stops moving after touching the gear shifting block 3. The cut-off needle seat 2 continues to move under the action of inertia and stops when it touches the gear shifting block 3, and at the same time, the cut-off needle seat locking hook 23 and the gear shifting block locking hook 311 are snapped together with each other.

A method of using an electric tube cutting needle of an embodiment of the present application is as follows: when the winding button 83 is pressed, the winding slider 1 is reset to snap together with the cut-off needle seat 2 and drives the cut-off needle seat 2 and the outer needle seat 4 to move in the direction opposite to the needle feed direction, thereby completing the winding; when the trigger linkage 5 is pressed, the outer needle seat 4 and the cut-off needle seat 2 are moved in the needle feed direction, wherein the outer needle seat 4 and the cut-off needle seat 2 are successively blocked by the gear shifting block 3, thereby completing the excitation.

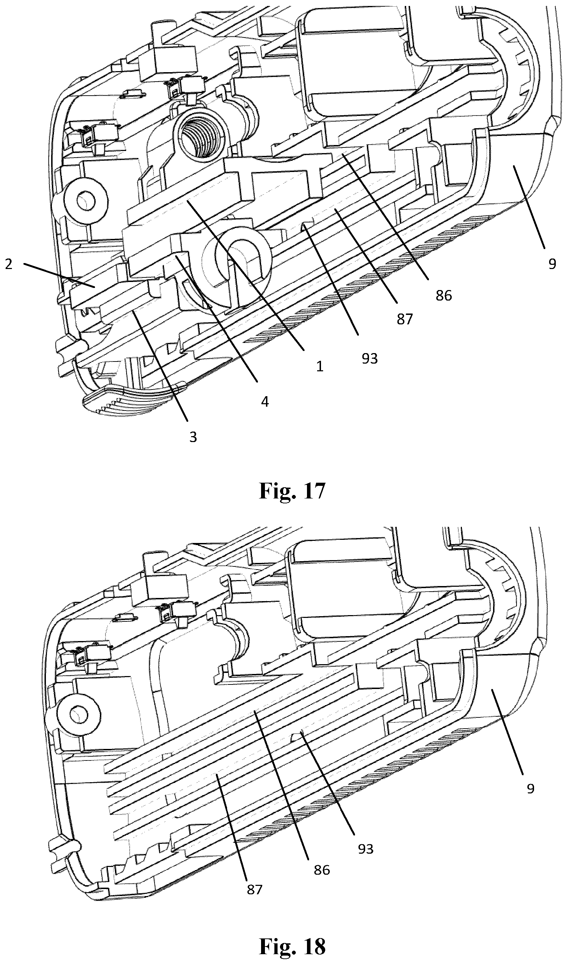

As shown in FIGS. 17 to 19, the housing has two parallel tracks, wherein the winding slider 1 is located on a first track 86, and the cut-off needle seat 2 and the outer needle seat 4 are located on a second track 87.

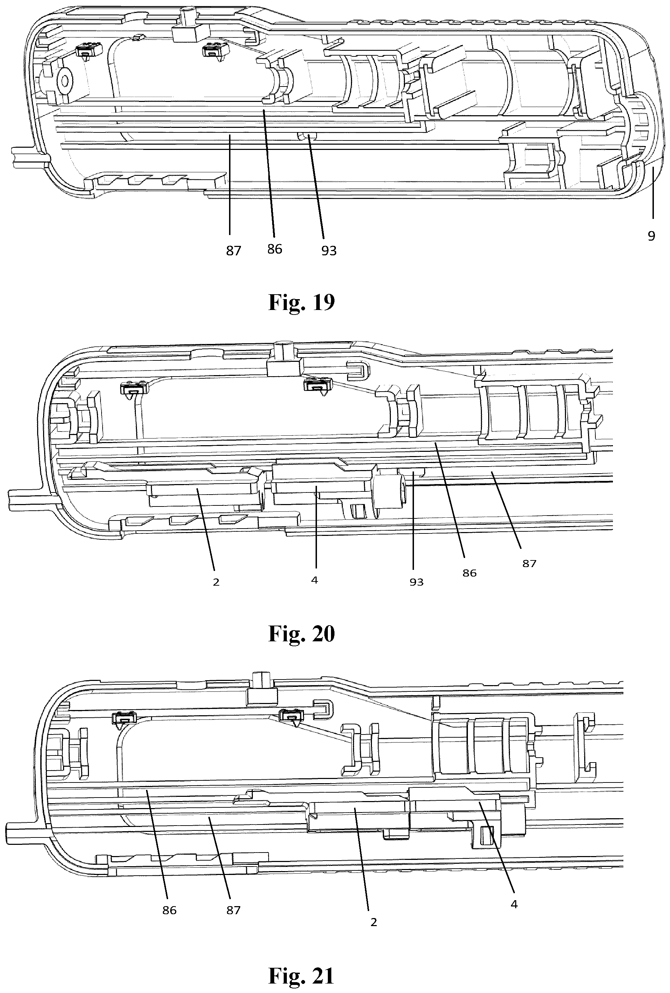

As shown in FIGS. 20 and 21, an indication window 93 is provided in the position on the lower housing 9 that corresponds to the second track 87 where the cut-off needle seat 2 and the outer needle seat 4 are located, and part of the cut-off needle seat 2 or the outer needle seat 4 within the track can be seen through the indication window, wherein the cut-off needle seat 2 and the outer needle seat 4 are arranged in different colors, so as to judge the conditions within the housing. FIG. 20 is a diagram of the positions of the cut-off needle seat 2 and the outer needle seat 4 after the excitation, and the outer needle seat 4 can be seen from the indication window 93. FIG. 21 is a diagram of the positions of the cut-off needle seat 2 and the outer needle seat 4 after winding, and the cut-off needle seat 2 can be seen from the indication window 93. When the cut-off needle seat 2 is seen, it means that the winding is completed and excitation can be performed.

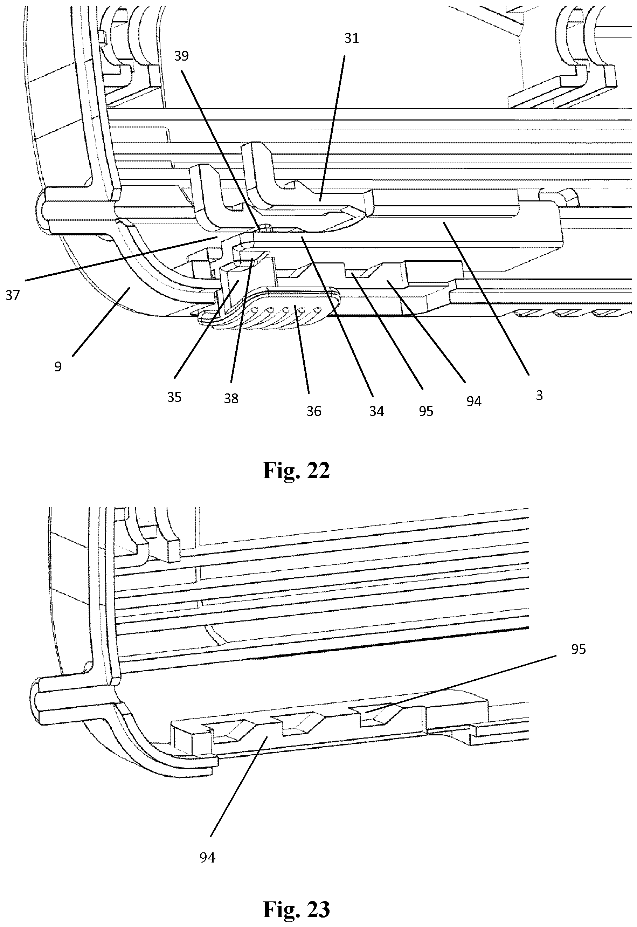

Referring to FIGS. 22 and 23, FIG. 2 is a structural schematic diagram of the connection of the gear shifting block with the housing. FIG. 23 is a structural schematic diagram of a part of the housing which is connected to the gear shifting block. In FIG. 22, the side of the gear shifting block 3 that is close to the housing extends in the needle feed direction to form a pressing portion 34, which is connected to the second pushbutton 36 disposed on the outer side of the housing by means of a connecting all 35. The housing is provided with a long through hole 94 for the movement of the second pushbutton 36. The pressing portion 34 and the front end of the blocking wall 31 are arranged as two separate portions, i.e. upper and lower portions, and a gap 37 is provided between the pressing portion 34 and the front end of the blocking wall 31. The two-separate-portion design of the pressing portion 34 and the front end of the blocking wall 31 is for making it easier to realize the elastic deformation, which is more easily realized when the second pushbutton 36 is pressed and the downwardly-pressing bevel 15 presses the gear shifting block 3. Trapezoidal protrusions 38 are respectively provided on two sides of the connection wall 35. A plurality of trapezoidal slots 95 whose shapes and sizes correspond to those of the trapezoidal protrusions 38 are arrayed longitudinally on two sides of the long through hole 94 in the housing, and there are three trapezoidal slots 95 in this embodiment. When no external force is applied on the second pushbutton 36, the trapezoidal protrusions 38 are located exactly in the trapezoidal slots 95. When a force is applied on the second pushbutton 36 from the inner side of the housing, trapezoidal protrusions 38 are disengaged from the trapezoidal slots 95, and the gear shifting block 3 can be pushed by the second pushbutton 36 to move parallel to the needle feed direction. In this way, the gear shifting block 3 is slidably arranged on the housing and positioned by the trapezoidal slot 95. In FIG. 22, the side surface of the trapezoidal projection 38 on the side of the needle feed direction is a vertical surface, and the other side is an inclined surface that is outwardly inclined. Due to the setting of the inclined surface of the trapezoidal protrusion 38 on the side opposite to the needle feed direction, it does not need to be pressed when toggling in the direction opposite to needle feed direction, and it needs to be pressed in order to shift the gear when toggling in the needle feed direction.

A gear shifting block projection 39 is provided on the back of the second pushbutton 36. After excitation, the cut-off needle seat 2 exactly abuts against the gear shifting block projection 39, so that gear shifting cannot be performed on a biopsy needle after excitation. After winding, the cut-off needle seat 2 leaves the position of the gear shifting block projection 39, and the second pushbutton 36 can be pressed for gear shifting. The gear shifting block projection 39 is established to prevent a doctor from incorrectly operating the gear after sampling, causing the inner needle and the cut-off needle to squeeze the tissue inside the outer needle, and can therefore only be used for gear shifting as needed after winding.

In the embodiments of the present application, the gear shifting method of the gear shifting block 3 is as follows: pushing the second pushbutton 36 in the direction opposite to the needle feed direction to shorten the stroke of the cut-off needle seat 2 and the outer needle seat 4; pressing the second pushbutton 36, and pushing the second pushbutton 36 in the needle feed direction to lengthen the stroke of the cut-off needle seat 2 and the outer needle seat 4.

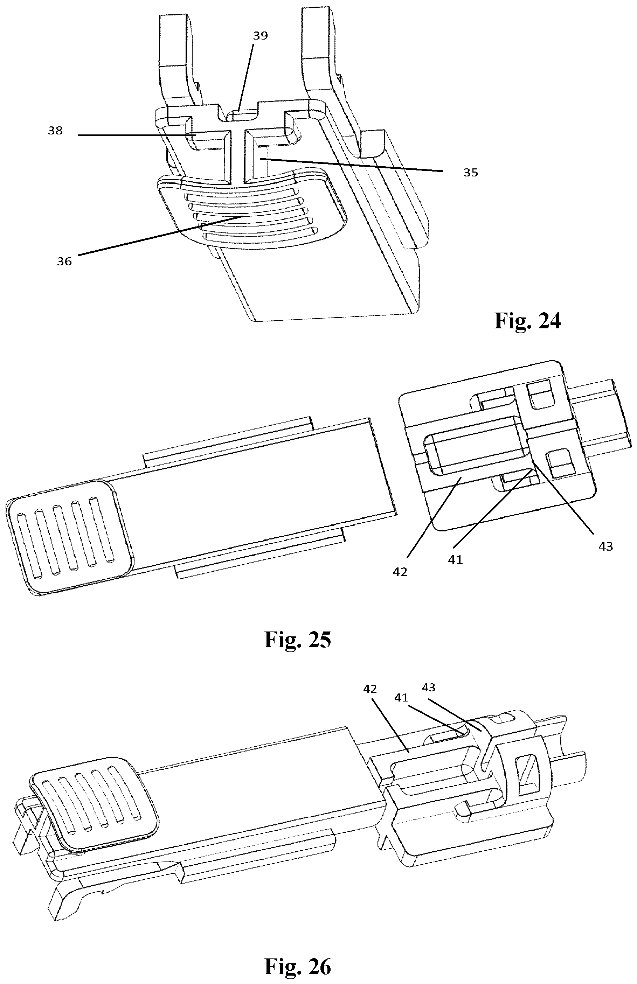

FIG. 24 is a structural schematic diagram of the gear shifting block 3 from another perspective according to an embodiment of the present application, from which it can be seen that the gear shifting block projection 39 is located at the back of the second pushbutton 36.

After excitation, the outer needle seat 4 and the cut-off needle seat 2 stop by impacting on the gear shifting block 3. Referring to FIG. 16, a recess 40 is provided in the slide groove 32 on the connecting surface of the gear shifting block 3 with the cut-off needle seat 2 and the outer needle seat 4. Referring to FIG. 11, the right end of the side of the cut-off needle seat 2 this is close to the gear shifting block 3 extends towards the gear shifting block3 to form an extension 24, and the size of the extension 24 corresponds to the size of the recess 40. After excitation, the cut-off needle seat 2 is moved in the needle feed direction, and the extension 24 is located in the groove 40. The extension 24 stops when it impacts on the wall of the recess 40, so as to avoid impacting the weak parts, such as the blocking hook of the cut-off needle seat 2.

The outer needle seat 4 and the cut-off needle seat 2 stop by impacting on the gear shifting block 3. Referring to FIGS. 25 to 27, when the outer needle seat 4 impacts on the gear shifting block 3, two arc corners 41, which are arranged on the outer needle seat 4, are located at the tailing of the rectangular tab 42 of the outer needle seat 4 that slides within the slide groove 32, and are located between the side surface of the rectangular tab 42 and the blocking wall 43 of the outer needle seat 4. The arc corners 41 impact the two walls of the slide groove 32 first when the outer needle seat 4 slides along the slide groove 32 of the gear shifting block 3. The jerky movement of the outer needle can be reduced by means of elastic energy-absorption in which the two walls of the slide groove 32 expand outwardly.

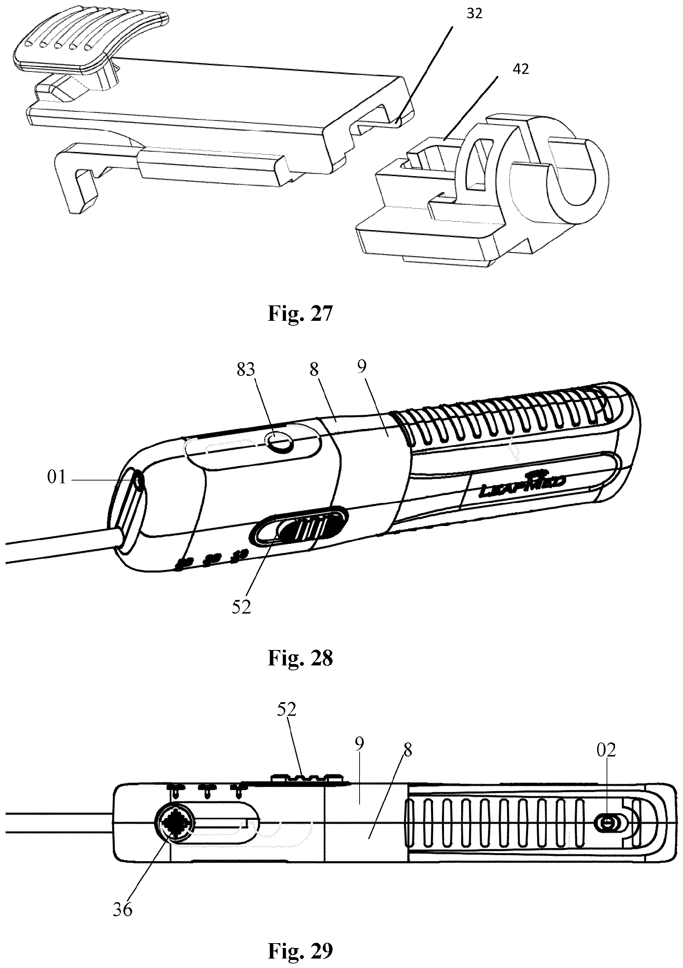

FIG. 28 is a schematic diagram of the external structure of an electric tube cutting needle according to another embodiment of the present application. FIG. 29 is a side schematic diagram of the external structure of an electric tube cutting needle according to another embodiment of the present application. As shown in FIGS. 28 and 29, this structure adds an illumination lamp 01, and a power switch 02 of the electric tube cutting needle is arranged at the tailing of the housing. The housing consists of an upper housing 8 and a lower housing 9 as previously described.

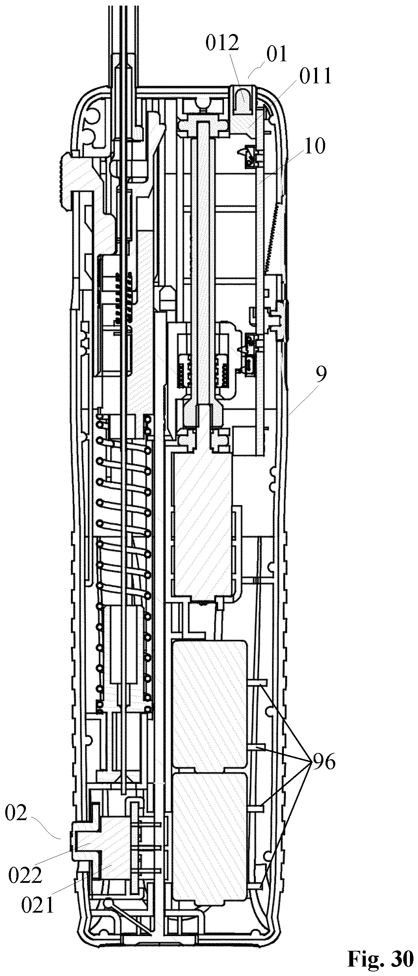

FIG. 30 is a sectional view of the electric tube cutting needle shown in FIG. 28. It is to be noted that although only the lower housing 9 is shown in FIG. 30, it can be understood that the first side/second side/first end/second end of the lower housing 9 may represent the first side/second side/first end/second end of the overall housing. As shown in FIG. 30, the illumination lamp 01 comprises a lamp cylinder 011, which is connected to the circuit board 10, and a lamp bead 012. Because of the vibration caused by the motor 7 when the electric tube cutting needle is used, the lamp bead 012 is arranged in the lamp cylinder 011 for the stability of the light. According to the viewing angle of FIG. 30, the right side is the first side of the housing, the left side is the second side of the housing, the upper end is the first end of the housing, the lower end is the second end of the housing, and the left-toward direction is the needle feed direction during the puncture surgery. As can be seen in conjunction with FIGS. 29 and 30, the power switch 02 is located away from the first pushbutton 52 and the winding button 83, which helps to prevent the power switch 02 from being accidentally touched during the surgical operation, especially when the first pushbutton 52 and the winding button 83 are pressed.

The power switch 02 is a toggle switch having a main body 021 and a toggle block 022 protruding from the main body 021. The main body 021 is located within the housing, and the top of the toggle block 022 being flush with the housing, so that accidental touching of the power switch 02 can be avoided as much as possible.

Furthermore, referring to FIG. 28, since the housing is flat as a whole, there is small space in the up and down direction (referring to FIG. 29), and a wire is arranged (the wire is not shown in the figure) close to the first side of the housing (referring to FIG. 30) to make full use of the inner space of the housing. The inner wall of the bottom surface of the lower housing 9 is provided with a plurality of, for example, four ribs 96. The battery 81 is located on top of the ribs 96, and there is a gap between the battery 81 and the inner wall of the bottom surface, thus forming a channel through which the wire passes and then extends upwards to the circuit board 10.

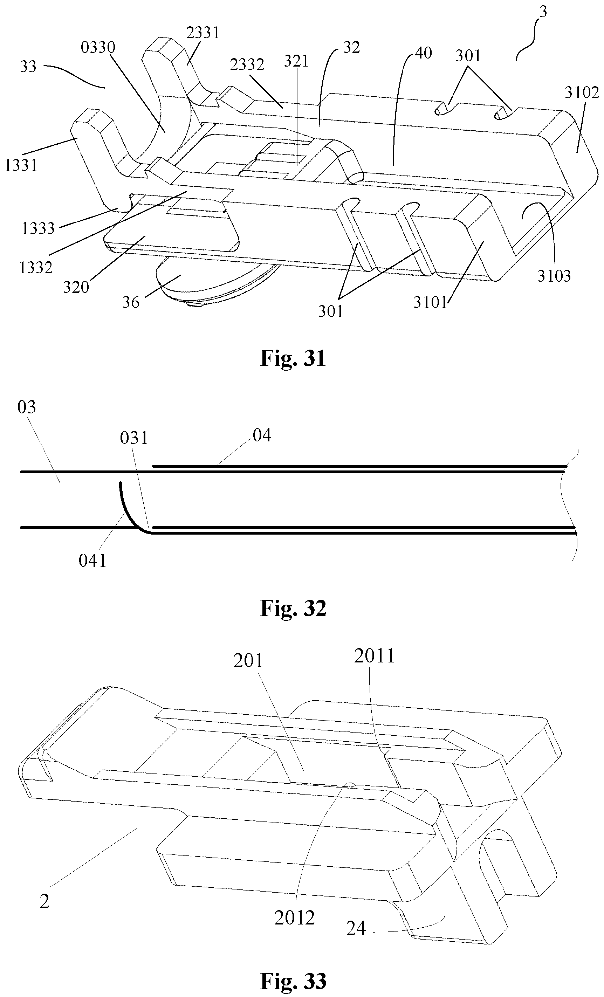

FIG. 31 is a structural schematic diagram of a gear shifting block in another embodiment of the present application. As shown in FIG. 31, the gear shifting block 3 has a blocking hook 33, a base plate 320, a slide groove 32, and a second pushbutton 36. The blocking hook 33 comprises a first arm 1331, a second arm 1332, a third arm 2331, a fourth arm 2332, and a connecting portion 0330. The base plate 320 may be regarded as being formed by extending the bottom 3103 of the slide groove 32. The second pushbutton 36 is located on a lower surface of the base plate 320 (at the viewing angle in the figure). The first arm 1331 is connected to the second arm 1332 in an L-shape, the third arm 2331 is connected to the fourth arm 2332 in an L-shape, and the corners of the two L-shapes are connected by a connecting portion 0330, thereby significantly enhancing the strength of the two L-shapes, so that the gear shifting block 3 remains intact when it is impacted by the cut-off needle seat 2.

A stopper 321 is provided in the slide groove 32 to provide a firmer impact surface to more securely withstand the direct impact from the cut-off needle seat 2. A plurality of wall grooves 301 are provided on the outer sides of the first wall 3101 and the second wall 3102 of the slide groove 32, and there are four wall grooves 301, as shown in FIG. 31. Considering the fact that when the cut-off needle seat 2 imposes an impact, the direction of the impact force is not absolutely parallel to the extension direction of the slide groove 32, i.e., there exists a transverse component, in this case, the wall grooves 301 can enable the first wall 3101 and the second wall 3102 to have a certain transverse flexibility that helps to prevent them from shattering under the impact from the cut-off needle seat 2.

A downward first protrusion and a downward second protrusion are respectively provided below the first arm 1331 and the third arm 2331. The first protrusion 1333 protruding in the direction of the base plate 320 is visible in the figure, while the second protrusion is not visible in the figure since it is sheltered. The protrusions below the first arm 1331 and the third arm 2331 are able to rest against the upper surface of the base plate 320 when the second arm 1332 and the fourth arm 2332 are bent downwardly to a certain extent, thereby avoiding damage caused by excessive downward bending of the second arm 1332 and the fourth arm 2332.

Furthermore, it is also possible to increase the thickness of the first wall 3101 and the second wall 3102 of the slide groove 32 to make it more resistant to impact from the cut-off needle seat 2. The thickness of the first wall 3101 and the second wall 3102 may be 4 to 6 times the thickness of the bottom 3103 of the slide groove.

The cut-off needle seat 2 and the outer needle seat 4 mentioned above can form a combined structure, which contains a limit portion. The limit portion enables mutual circumferential and axial limit to be formed between the outer needle 03 and the cut-off needle 04. This is further described below.

FIG. 32 is a schematic diagram of an outer needle and part of a tip of a cut-off needle of an electric tube cutting needle according to an exemplary embodiment of the present application. As shown in FIG. 32, the outer needle 03 is provided with a side hole 031, and the cut-off needle 04 is provided with a cut-off piece 041 at the end thereof. After excitation, the outer needle 03 is moved and then stops, and the cut-off needle 04 continues to move and causes the cut-off piece 041 to enter into the outer needle 03 through the side hole 031 so as to cut off the tissue accommodated in the outer needle 03.

According to the above operation mode, the cut-off piece 041 is required to not only able to align with the side hole 031 but also have a moderate length of entry into the side hole 031, because it is difficult to cut off the tissue smoothly if the entry length is too short and it is easy to lead to the breakage of the cut-off piece 041 if the entry length is too long. Therefore, for the position of the cut-off piece 041, both circumferential and axial positional accuracy is required.

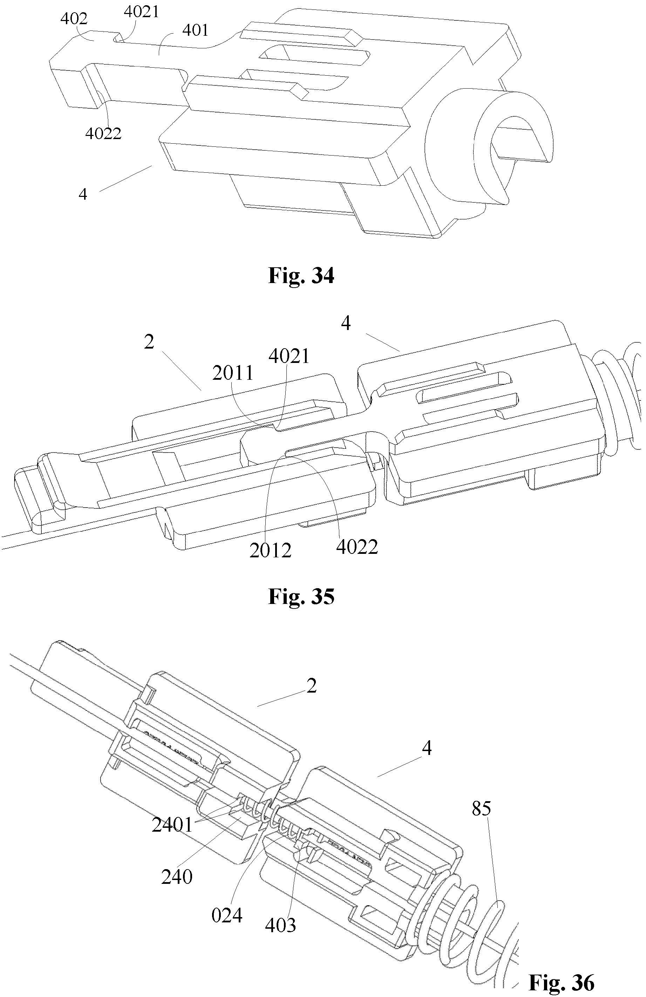

To this end, in the embodiments of the present application, in order to further improve the position accuracy of the cut-off piece, the cut-off needle seat 2 and the outer needle seat 4 can be formed into a combined structure, as shown in FIGS. 33 to 35. FIG. 33 is a structural schematic diagram of the cut-off needle seat of an electric tube cutting needle according to another exemplary embodiment of the present application. FIG. 34 is a structural schematic diagram of the outer needle seat of an electric tube cutting needle according to another exemplary embodiment of the present application. FIG. 35 is a schematic diagram of a combined structure formed by the cut-off needle seat and the outer needle seat of an electric tube cutting needle according to an exemplary embodiment of the present application;

The cut-off needle seat 2 has a groove body 201 recessed downwardly from its upper surface, and the groove body 201 extends along a longitudinal direction, which is the travel direction of the cut-off needle seat 2 (the left-toward direction in the figure). A transverse end face (see FIG. 35) of the groove body 201 that is close to the outer needle seat 4 forms a blocking surface. Since the right end of the groove body 201 is open, the blocking surface here consists two parts, i.e., a first blocking surface 2011 and a second blocking surface 2012 in the figure.

A longitudinally extending limit lever 401 is connected to the side of the outer needle seat 4 that is close to the cut-off needle seat 2 (i.e., the left side in the figure, see FIG. 35). A limit block 402 is connected to the end of the limit 401. The width of the limit block 402 is close to the width of the groove body 201 (see FIG. 35), so that the limit block 402 is unable to rotate along the left-right direction in the figure as an axis. It can be seen that this limits the circumferential rotation of the outer needle 03 and the cut-off needle 04 to each other, forming a circumferential positioning, which helps the above-mentioned cut-off piece 041 to align with the side hole 031.

The width of the limit block 402 is greater than the width of the limit lever 401, so that a first abutting surface 4021 and a second abutting surface 4022 are formed on the side of the limit block 402 facing the outer needle seat 4 (i.e., the right side in the figure). The above-mentioned cut-off needle seat 2 and the outer needle seat 4 are suitable for the two structures of the gear shifting block 3 mentioned above. As can be seen from the description above, the gear shifting block 3, upon impact, can cause some uncertainty as to where the cut-off needle seat 2 stops after being blocked by the gear shifting block 3, due to the resilience of the blocking hook 33 at its end (see FIG. 8) or the first arm to the fourth arm 1331,1332,2331,2332 (see FIG. 31).

With the structure shown in FIGS. 33 to 35, by selecting a suitable length of the limit lever 401, when the extension 24 of the cut-off needle seat 2 impacts the stopper 321 of the gear shifting block 3, the first abutting surface 4021 and the second abutting surface 4022 abut against the first blocking surface 2011 and the second blocking surface 2012 mentioned above, respectively. That is to say, the limit block 402 is also impacted while the stopper 321 is impacted. In this case, the limit lever 401 is subjected to a tensile force. It is difficult for the limit lever 401 to deform under the action of the tensile force, so that the position of the cut-off needle seat 2 at the time of stopping is deterministic, and the accuracy of the length of the cut-off piece 041 into the side hole 031 is thereby ensured.

A spring may be provided between the cut-off needle seat 2 and the outer needle seat 4, as shown in FIG. 36. FIG. 36 is a schematic diagram of a spring provided between the cut-off needle seat and the outer needle seat according to an exemplary embodiment of the present application. The spring is a needle seat spring 024 in the figure, which is stuck between the cut-off needle seat 2 and the outer needle seat 4. The specific setting can be as follows: a seat notch 240 is provided in the extension 24 of the cut-off needle seat 2, and one end of the needle seat spring 024 is pressed against the end surface 2401 of the seat notch 240; the lower surface of the outer needle seat 4 is provided with a downwardly-extending convex rib 403, and the other end of the needle seat spring 024 is pressed against the side of the convex rib 403 facing the cut-off needle seat 2.

The needle seat spring 024 is capable of bringing about beneficial effects in various aspects, which are analyzed and described below.

After excitation, the cut-off needle seat 2 is pushed by the outer needle seat 4. After the outer needle seat 4 stops, the cut-off needle seat 2 continues to advance under inertia and the cut-off needle 04 cuts off the tissue. Due to the presence of friction, the speed and force with which the cut-off needle 04 finally cuts off the tissue cannot be stable to meet the expected requirements, and sometimes may not be able to cut off the tissue

After the needle seat spring 024 is added, because the elastic force of the needle seat spring 024 is less than the elastic force of the spring 85 that provides the excitation power, the needle seat spring 024 is ejected in the direction of the cut-off needle seat 2, so the cut-off needle seat 2 advances under the action of the elastic force provided by the needle seat spring 024 in addition to the action of inertia. That is to say, the needle seat spring 024 can additionally provide a cut-off force for the cut-off needle 04 to ensure that the tissue can be cut off.

At the same time, the cut-off needle seat 2 impacts on the blocking hook 33 of the gear shifting block 3 (see FIG. 8) (see FIG. 31) after excitation or rebounds after impact, and all the energy of elastic force is borne by the gear shifting block locking hook 311 and the cut-off needle seat locking hook 23 (see FIG. 8), which is easy to cause damage to the gear shifting block locking hook 311 and the cut-off needle seat locking hook 23. With the addition of the needle seat spring 024, the outward elastic force thereof can provide a certain resistance to the rebound of the cut-off needle seat 2, thereby reducing the impact force between the gear shifting block locking hook 311 and the cut-off needle seat locking hook 23, and helping to maintain their firmness when they are hooked to each other.

In addition, the needle seat spring 024, as an energy storage component, has a certain buffering effectiveness, and can absorb part of the axial vibration of the cut-off needle 04 after the cut-off needle 04 is excited, improving the stability of the cut-off needle 04, thereby improving the overall stability of the needle tip to a certain extent, and reducing the needle tip jitter.

The above specific embodiments do not constitute a limitation on the scope of protection of the present application. It should be appreciated by those skilled in the art that a wide variety of modifications, combinations, sub-combinations and substitutions can occur depending on design requirements and other factors. Any modifications, equivalent substitutions, improvements, etc. made within the spirit and principle of the present application shall be included in the scope of protection of the present application.

### List of symbols

01 illumination lamp; 011 lamp cylinder; 012 lamp bead; 02 power switch; 021 main body; 022 toggle block; 03 outer needle; 04 cut-off needle; 1 winding slider; 2 cut-off needle seat; 3 gear shifting block; 4 outer needle seat; 5 trigger linkage; 6 screw rod; 7 motor; 8 upper housing; 9 lower housing; 10 circuit board; 11 first protrusion; 12 connection hole; 13 first longitudinal groove; 14 tubular structure; 15 downward-pressing bevel; 16 bottom edge; 21 second protrusion; 211 unlocking bevel; 22 second longitudinal groove; 23 cut-off needle seat locking hook; 24 extension; 31 blocking wall of gear shifting block; 311 gear shifting block locking hook; 32 slide groove; 33 blocking hook; 331 blocking hook bevel; 332 blocking hook top surface; 34 pressing portion; 35 connecting wall; 36 second pushbutton; 37 gap; 38 trapezoidal protrusion; 39 gear shifting block protrusion; 40 recess; 41 arc corner; 42 rectangular tab; 43 blocking wall of outer needle seat; 51 trigger button; 52 first pushbutton; 61 plastic screw rod; 62 metal shaft; 71 rotary shaft; 81 battery; 82 indicator lamp; 83 winding button; 84 display hole; 85 spring; 86 first track; 87 second track; 88 first bearing; 89 second bearing; 90 inner needle seat; 91 front contact switch; 92 rear contact switch; 93 indicator window; 94 long through hole; 95 trapezoidal slot; 96 rib; 1331 first arm; 1332 second arm; 2331 third arm; 2332 fourth arm; 0330 connecting portion; 320 base plate; 321 stopper of slide groove; 3103 bottom of slide groove; 3101 first wall; 3102 second wall; 301 wall groove; 1333 first protrusion; 031 side hole; 041 cut-off piece; 201 groove body; 2011 first blocking surface; 2012 second blocking surface; 401 limit lever ; 402 limit block; 4021 first abutting surface; 4022 second abutting surface; 024 needle seat spring; 240 notch; 2401 end face; 403 convex rib.

## Claims

1. An illumination assembly for an electric tube cutting needle, the electric tube cutting needle comprising a puncture biopsy needle assembly (03,04), a battery (81), a motor (7), a housing (8,9), a first pushbutton (52) for the excitation of a puncture needle, and a winding button (83) for winding, the puncture biopsy needle assembly (03,04) piercing from a first end of the housing (8,9), **characterized in that** the illumination assembly comprises an illumination lamp (01), a circuit board (10), and a power switch (02), wherein:
the housing is formed by an upper housing (8) and a lower housing (9) snapped together, and extends as a whole in the needle feed direction, and the illumination lamp (01) is located at the first end of the housing (8,9) and is electrically connected to the circuit board (10);
the first pushbutton (52) is located at a bottom of the upper housing (8) and close to the first end of the housing (8,9), and the winding button (82) is located at a first side of the housing (8,9) and close to the first end of the housing (8,9), wherein the first side of the housing (8,9) is formed by split-joining of a first side of the upper housing (8) and a first side of the lower housing (9);
the power switch (02) is located on a second side of the housing (8,9) and close to a second end of the housing (8,9), wherein the second side of the housing (8,9) is a side opposite to the first side of the housing (8,9), and the second end of the housing (8,9) is an end opposite to the first end of the housing (8,9).

2. A gear shifting assembly for an electric tube cutting needle, **characterized by** comprising a gear shifting block (3) and a second pushbutton (36), wherein:
the gear shifting block (3) comprises a blocking hook (33), a base plate (320) and a slide groove (32),
the blocking hook (33) comprises a first arm (1331), a second arm (1332), a third arm (2331), a fourth arm (2332), and a connecting portion (0330);
the base plate (320) is connected to and coplanar with the bottom of the slide groove (32);
the first arm (1331) is connected to the second arm (1332) in an L-shape, the second arm (1332) is connected to the top edge of the first wall (3101) of the slide groove (32), and the second arm (1332) extends in the same direction as the first wall (3101);
the third arm (2331) is connected to the fourth arm (2332) in an L-shape, the fourth arm (2332) is connected to the top edge of the second wall (3102) of the slide groove (32), and the second arm (2332) extends in the same direction as the second wall (3102);
two ends of the connecting portion (0330) are connected to the junction between the first arm (1331) and the second arm (1332) and the junction between the third arm (2331) and the fourth arm (2332) respectively, so that the connecting portion (0330), the first arm (1331) and the third arm (2331) form a U-shape, the first arm (1331) and
the third arm (2331) extending in a direction away from the base plate (320); and
the second pushbutton (36) is provided on a lower surface of the base plate (320), and
the pressing direction of the second pushbutton (36) is the same as the extension direction of the first arm (1331) and the third arm (2331).

3. The gear shifting assembly according to claim 2, **characterized in that** the gear shifting block (3) further comprises a recess (40) for accommodating the travel of the cut-off needle seat (2);
the slide groove (32) is provided with a stopper (321) therein, which is adjacent to the end of the recess (40) that is located inside the slide groove (32), so that the side of the stopper (321) that faces an inlet of the slide groove (32) is used to receive the impact from the cut-off needle seat (2) when travelling.

4. The gear shifting assembly according to claim 2 or 3, **characterized in that** a first protrusion (1333) is provided at the junction between the first arm (1331) and the second arm (1332);
a second protrusion is provided at the junction between the third arm (2331) and the fourth arm (2331); and
the first protrusion (1333) and the second protrusion both protrude towards the base plate (320).

5. An electric tube cutting needle, comprising: a housing, a tube cutting needle mechanical unit and a tube cutting needle control unit, wherein
the interior of the housing has a first track (86) and a second track (87) arranged in parallel;
the tube cutting needle mechanical unit comprises:
a puncture biopsy needle assembly comprising an inner needle, an outer needle (03), and a cut-off needle (04);
a needle seat component comprising a cut-off needle seat (2), an outer needle seat (4) and an inner needle seat (90) disposed on the second track (87), the cut-off needle seat (2), the outer needle seat (4) and the inner needle seat (90) being connected to the inner needle, the outer needle (03) and the cut-off needle (04), respectively, and the cut-off needle seat (2), the outer needle seat (4) and the inner needle seat (90) being distanced from a needle exit end sequentially from near to far, the inner needle seat (90) being fixedly connected to the housing, a spring (85) being provided between the inner needle seat (90) and the outer needle seat (4);
a winding assembly for winding the needle seat component, comprising a winding slider (1) disposed on the first track (86), the winding slider (1) being provided so as to be capable of driving the cut-off needle seat (2) and the outer needle seat (4) in the direction opposite to the needle feed direction to a winding position;
an excitation assembly for excitation of the cut-off needle seat (2) and the outer needle seat (4) in the winding position;
a gear shifting assembly for adjusting an excitation gear of the electric tube cutting needle;
the tube cutting needle control unit comprises:
a motor drive module for driving the winding assembly and the excitation assembly to realize winding and excitation reset;
a detection module for detecting the position of the winding slider (1);
a control module for controlling the operation of the motor drive module according to the detection result of the detection module.

6. The electric tube cutting needle according to claim 5, **characterized in that** the winding assembly is arranged such that:
when the winding slider (1) is in a first position, the winding slider (1) can be moved in the needle feed direction under the drive by the motor drive module, such that a connection hole (12) at the end of the winding slider (1) snaps together with the second protrusion (21) at the end of the cut-off needle seat (2);
when the winding slider (1) is moved to a second position, the winding slider (1) can be moved in the direction opposite to the needle feed direction under the drive by the motor drive module, such that the cut-off needle seat (2) and the outer needle seat (4) are moved along with the winding slider (1) in the direction opposite to the needle feed direction and such that the spring (85) is compressed to store energy;
when the winding slider (1) is again in the first position, the winding slider (1) stops moving.

7. The electric tube cutting needle according to claim 5, **characterized in that** the gear shifting assembly is a gear shifting assembly according to any one of claims 2 to 4.

8. The electric tube cutting needle according to claim 5, **characterized in that** the excitation assembly comprises: a trigger linkage (5), a trigger button (51) disposed at the rear end of the trigger linkage (5), and a first pushbutton (52) connected to the trigger linkage (5), wherein
when the trigger button (51) is pressed or the first pushbutton (52) is pushed, the trigger linkage (5) is moved in the needle feed direction, and the end of the trigger linkage (5) is moved along a ramp-like surface of an unlocking bevel (211) on the cut-off needle seat (2), thereby separating the winding slide (1) from the cut-off needle seat (2); then the cut-off needle seat (2) and the outer needle seat (4) are moved in the needle feed direction under the action of the elastic force of the spring (85); the outer needle seat (4) stops moving when it touches the gear shifting block (3), the cut-off needle seat (2) continues moving under the action of inertia and stops when it touches the gear shifting block (3), and at the same time, the cut-off needle seat locking hook (23) on the cut-off needle seat (2) and the gear shifting block locking hook (311) on the gear shifting block (3) snap together with each other.

9. The electric tube cutting needle according to claim 5, **characterized in that** the motor drive module comprises a motor (7) and a screw rod (6), wherein the motor (7) is provided to be capable of performing forward or reverse rotation, thereby driving the screw rod (6) to perform forward or reverse rotation, which in turn pushes the winding slider (1) to move in the needle feed direction or in the direction opposite to the needle feed direction.

10. The electric tube cutting needle according to claim 5, **characterized in that** the detection module comprises: a front contact switch (91) and a rear contact switch (92), wherein
the rear contact switch (92) is arranged to emit a first detection signal when the rear contact switch (92) comes into contact with the first protrusion (11) on the winding slider (1);
the front contact switch (91) is arranged to emit a second detection signal when the front contact switch (91) comes into contact with the first protrusion (11) on the winding slider (1).

11. The electric tube cutting needle according to claim 10, **characterized in that** the control module comprises a circuit board (10), the circuit board (10) is arranged such that:
if the circuit board (10) receives the first detection signal, the circuit board (10) controls the motor drive module to drive the winding slider (1) to move in the needle feed direction;
if the circuit board (10) receives the second detection signal, the circuit board (10) controls the motor drive module to drive the winding slider (1) to move in the direction opposite to the needle feed direction.

12. The electric tube cutting needle according to claim 11, **characterized in that** it further comprises an illumination assembly according to claim 1.

13. The electric tube cutting needle according to claim 5, **characterized in that** the cut-off needle seat (2) has a groove body (201) recessed downwardly from its upper surface, the groove body (201) extends along a longitudinal direction, which is the direction of travel of the cut-off needle seat (2), and the transverse end face of the groove body (201) that is close to the outer needle seat (4) forms a blocking surface (2011, 2012);
a longitudinally extending limit lever (401) is connected to the side of the outer needle seat (4) that is close to the cut-off needle seat (2), and a limit block (402) is connected to the end of the limit lever (401), the limit block (402) being unable to rotate about the longitudinal direction taken as an axis within the groove body (201), thereby constituting a circumferential limit;
a portion of the limit lever (401) and the limit block (402) are located within the groove body (201), the limit block (402) has a width greater than the width of the limit lever (401), and the limit lever (401) has such a length that while the extension (24) of the cut-off needle seat (2) impacts the gear shifting block (3) of the electric tube cutting needle, the side of the limit block (402) that faces the outer needle seat (4) abuts against the blocking surface (2011,2012).

14. The electric tube cutting needle according to claim 5, **characterized in that** the electric tube cutting needle further comprises a needle seat spring (024), which is arranged to be sleeved on the cut-off needle (04) and stuck between the cut-off needle seat (2) and the outer needle seat (4); the needle seat spring (024) is a compression spring having an elastic force less than that of the spring (85) that provides excitation for the electric tube cutting needle.

15. The electric tube cutting needle according to claim 14, **characterized in that** the end of the extension (24) of the cut-off needle seat (2) that faces the outer needle seat (4) is provided with a notch (240), and the first end of the needle seat spring (024) abuts against an end face (2401) of the notch (240), the end face (2401) being perpendicular to the cut-off needle (04);
the lower surface of the outer needle seat (4) has a downwardly extending convex rib (403), and the second end of the needle seat spring (024) abuts against the side surface of the convex rib (403) that faces the cut-off needle seat (2).
